Europäisches Patentamt

European Patent Office

Office européen des brevets

(11) Publication number: **0 315 768**
**A2**

## (12) EUROPEAN PATENT APPLICATION

(21) Application number: 88116001.4

(22) Date of filing: 28.09.88

(51) Int. Cl.⁴ **A61N 1/08** , **A61N 1/36** ,
**A61B 5/05**

(30) Priority: 07.10.87 PL 268120

(43) Date of publication of application:
**17.05.89 Bulletin 89/20**

(84) Designated Contracting States:
**DE FR IT**

(71) Applicant: **OSRODEK
BADAWCZO-ROZWOJOWY
ELEKTRONICZNEJ APARATURY MEDYCZNEJ
ul. Wolnosci 345a
41-800 Zabrze(PL)**

(72) Inventor: **Galecka, Jerzy
ul. Struzika 12 a/27
Zabrze(PL)**
Inventor: **Dolnicki, Jerzy
ul. Ks. Pospiecha 5a/8
Zabrze(PL)**

(74) Representative: **Ritter und Edler von Fischern,
Bernhard,Dipl.-Ing. et al
HOFFMANN - EITLE & PARTNER
Arabellastrasse 4
D-8000 München 81(DE)**

(54) **Output circuit of a stimulator.**

(57) An output circuit of a stimulator, generating bipolar pulses of current, finding application in diagnosis and therapy in medicine. The invention consists in applying, in the output circuit of a stimulator, one voltage-controlled current generator /5/ being switched on between the first bus-bar /6/ and a bridge comprising four electronic keys /1/, /2/, /3/ and /4/, which is with the opposite end connected to the other bus-bar /7/. The two remaining points /9/ and /10/ of the bridge constitute the output of the stimulator, whereto the impedance /13/ of a patient is connected.

The appropriate sequence of the control run as set up from a control system /11/ makes a two-directional pulse of current flow through the impedance of a patient.

Such a solution ensures preferable symmetry of the output pulse with regard to the shape and load of the both phases, and its stability in time as well as it facilitates the process of aligning the stimulator.

## Output circuit of a stimulator

### Technical field

The subject of the invention is an output circuit of a stimulator, generating bipolar pulses of current, to be applied in diagnosis and in therapy in medicine.

### Prior art statement

There are known solutions of stimulator output circuits generating bipolar pulses of current, for instance from the Polish specification No. 91456 and from the US specification No. 3,924,641.

The first solution comprises two current generators having the two terminals in common, coupled to one bus-bar, while the others provide a stimulator output being connected to load impedance. The output terminals are being alternately keyed to the other bus-bar, what enables the current flow through the load in both directions.

The other solution comprises current generators connected in series with two electronic keys and further on connected to the first bus-bar, the other terminals of bus-bars being the output of a stimulator, and via two keys being coupled to the other bus-bar.

Subsequent switching on the alternate pairs of keys makes the current flow through the load impedance in two directions.

In the hitherto-known solutions the attainment of symmetry of shape and symmetry of load of the both halves of the pulse, being necessary for preferable applications, requires precise aligning of current generators in the two branches of the output circuit. It makes the operation of aligning significantly more difficult, and it does not ensure a long-term stability of the output pulse symmetry.

### Purpose of the invention

In order to attain a preferable symmetry of shape and load of the output pulse and the simplicity of aligning in the output circuit, there was applied one, voltage-controlled, current generator co-operating with a bridge comprising four electronic keys.

### Nature of the invention

In the output circuit of the stimulator as per this present invention, the symmetry of the bipolar pulse of current is ensured by the application of one, voltage-controlled, current generator.

The current generator is switched on between the first bus-bar and connected into the transition point of input of the two keys, the outputs of which, being the output of the stimulator, join the output of the two subsequent keys. Their inputs join the other bus-bar. Due to operation of signals controlled by impedance of a patient, there occurs a flow of the two-directional pulses of current, being symmetrical as far as the shape and also the load of the both halves is concerned. Such a solution of the output circuit ensures minimum interference in recording action potentials during stimulation and minimum of electrochemical phenomena in electrodes as well as it facilitates the process of aligning of the stimulator.

### Example of embodiment

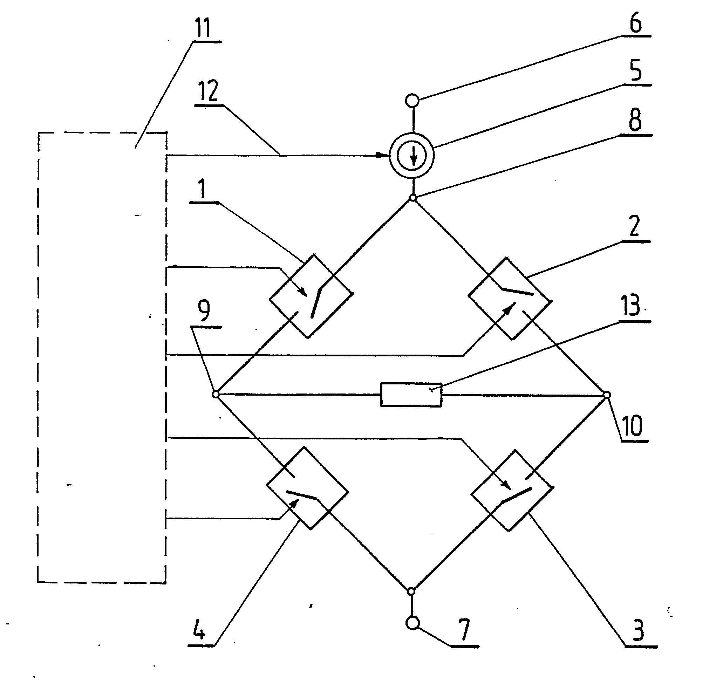

The subject of the invention will be disclosed in more detail on the basis of the example of embodiment as shown in the drawing, which is being a block diagram of its preferable practical realization.

A current generator 5 is switched on between the first bus-bar 6 and a transition point 8 of the inputs of electronic keys 1 and 2. The outputs 9 and 10 of the said keys, being at the same time the outputs of the stimulator, are connected to the outputs of keys 4 and 3, and their inputs are switched on into the second bus-bar 7. The current generator 5 and electronic keys 1, 2, 3 and 4 are connected to a control system 11.

### Functioning of the invention

The output circuit is being activated out of the control system 11, which, for the time of pulse, sets the amplitude of the output current by means of the rate of voltage 12. At the same time, for the pre-set time interval, there are switched on the electronic keys 1 and 3 in parallel branches of bridge, and then the electronic keys 2 and 4 in the remaining two branches.

In the first phase of a pulse, current flows from the bus-bar 6 through the current generator 5, key 1, output point 9, impedance 13 of a patient, output point 10, key 3 to the bus-bar 7.

In the second phase of a pulse, current flows from the bus-bar 6 through the current generator 5, key 2, output point 10, impedance 13 of a patient, output point 9, key 4 to the bus-bar 7. It can be noticed that the direction of the current flow through the impedance 13 of a patient in the second phase of a pulse is contrary to that in the first phase, and at ensuring the stability of amplitude of the current being generated by the current generator 5 and at precise countdown of the duration time of the both phases of the pulse, there is preserved the symmetry of shape and the zero resultant load, flowing through the impedance 13 of a patient.

Preferred utilization of the invention

The solution as per this present invention can be applied in all output circuits of stimulators, particularly of a cardiac muscle, where it is important to satisfy the condition of zero resultant load, flowing through the impedance of a tissue, at preserving the short duration time of the output pulse.

## Claims

An output circuit of a stimulator, particularly for biomedical application, activated by a control system comprising a controlled current generator and four electronic keys, characterized in that a current generator /5/ is switched on between the first bus-bar 6/, and the input of the two keys /1/ and /2/, being connected in a transition point 8/, the outputs /9/ and /10/ of the said keys being the output of the stimulator, join the outputs of the two subsequent keys /4/ and /3/, and the inputs of the said keys join the other bus-bar /7/.